# EUROPEAN PATENT APPLICATION

(11) **EP 4 524 174 A1**
(43) Date of publication of application: **19.03.2025**
(21) Application number: 23803193.4
(22) Date of filing: 01.02.2023
(51) Int. Cl.: C08G 59/66, C07C 323/12, C08G 75/045, C08K 5/17, C08K 5/37, C08L 63/00, C09J 163/00, C09K 3/10

(54) **CURING AGENT AND USE THEREOF**

(30) Priority: 10.05.2022 JP 2022077481; 24.08.2022 JP 2022132880
(71) Applicant: Shikoku Chemicals Corporation, Marugame-shi, Kagawa 763-8504 (JP)
(72) Inventor: SHIOIRI, Ryosuke, Ayauta-gun, Kagawa 769-0202 (JP); AOKI, Kazunori, Ayauta-gun, Kagawa 769-0202 (JP); KUMANO, Takeshi, Ayauta-gun, Kagawa 769-0202 (JP)
(74) Representative: Müller-Boré & Partner Patentanwälte PartG mbB
(86) International application number: PCT/JP2023/003137
(87) International publication number: WO 2023/218702

(57) **Abstract**

The present invention provides a curing agent containing a thiol compound represented by formula (I) and use thereof. Specifically, the present invention provides a curing agent containing a thiol compound represented by formula (I), a resin composition containing the curing agent and an epoxy compound, a resin composition containing the curing agent and an ene compound having a carbon-carbon double bond in the molecule, and adhesives and sealants containing these resin compositions. The present invention relates to a curing agent containing a thiol compound represented by formula (I), a resin composition containing the curing agent, an epoxy compound, and the like, and use of the resin composition.

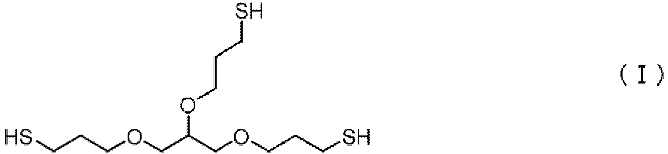

## Description

### Technical Field

The present invention relates to a curing agent containing a thiol compound and use thereof.

### Background Art

Compounds having multiple thiol groups in their molecule are well known as useful curing agents for epoxy resins. For example, an epoxy resin composition has been proposed that contains a polythiol compound as a curing agent and also contains a reaction product of an amine and an epoxy compound as a curing accelerator. This epoxy resin composition is said to have a long pot life and cure rapidly at relatively low temperatures (see PTL 1) .

Another epoxy resin composition has been proposed that contains, as a curing accelerator, a reaction product of an isocyanate compound having one or more isocyanate groups in the molecule and a compound having one or more primary and/or secondary amino groups in the molecule. This epoxy resin composition is also said to have a long pot life and excellent curability (see PTL 2).

Additionally, it is known that sulfur compounds containing an ether bond in their structure can give a cured resin product with excellent properties such as flexibility and moisture resistance when used as an epoxy-resin curing agent. On the other hand, when used as an adhesive, such sulfur compounds are unsatisfactory in adhesive strength on the adherend due to the flexibility of the structure (see PTL 3).

### Citation List

### Patent Literature

PTL 1: JPH06-211969A
PTL 2: JPH06-211970A
PTL 3: WO2016/171072A

### Summary of Invention

### Technical Problem

An object of the present invention is to provide a curing agent containing a thiol compound and use of the curing agent. Specifically, the present invention aims to provide a curing agent containing a specific thiol compound, a resin composition containing the curing agent and an epoxy compound, a resin composition containing the curing agent and an ene compound having a carbon-carbon double bond in the molecule, and adhesives and sealants containing these resin compositions.

### Solution to Problem

As a result of intensive research aimed at achieving the object, the present inventors found that a specific trithiol compound is useful as a curing agent, and that a resin composition containing the curing agent and a predetermined resin (an epoxy compound or an ene compound having a carbon-carbon double bond in the molecule) is useful as an adhesive and a sealant. The inventors conducted further research based on the findings and completed the present invention.

Specifically, the first invention is a curing agent comprising a thiol compound represented by formula (I).

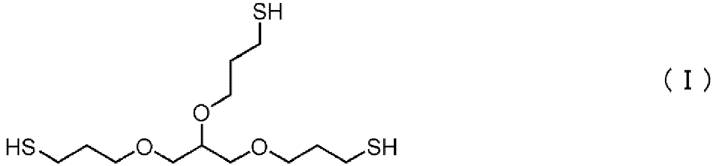

The second invention is a resin composition comprising the curing agent of the first invention and an epoxy compound.

The third invention is the resin composition of the second invention, further comprising an amine as a curing accelerator.

The fourth invention is a resin composition comprising the curing agent of the first invention and an ene compound having a carbon-carbon double bond in the molecule.

The fifth invention is an adhesive comprising the resin composition of any one of the second to fourth inventions.

The sixth invention is a sealant comprising the resin composition of any one of the second to fourth inventions.

The seventh invention is a cured product of the resin composition of any one of the second to fourth inventions.

### Advantageous Effects of Invention

The curing agent of the present invention contains a thiol compound represented by formula (I) and is usable as a curing agent for various resins.

The cured product of a resin composition containing the curing agent has a decreased crosslink density and thus can exhibit improved toughness. Therefore, the resin composition used as an adhesive can provide improved adhesive strength, which has been an issue in the art. Therefore, by using such a resin composition as a component, the present invention can provide adhesives and sealants having particularly excellent adhesive strength.

Additionally, due to its low viscosity, the curing agent of the present invention has improved workability during molding when used in a resin composition.

### Brief Description of Drawings

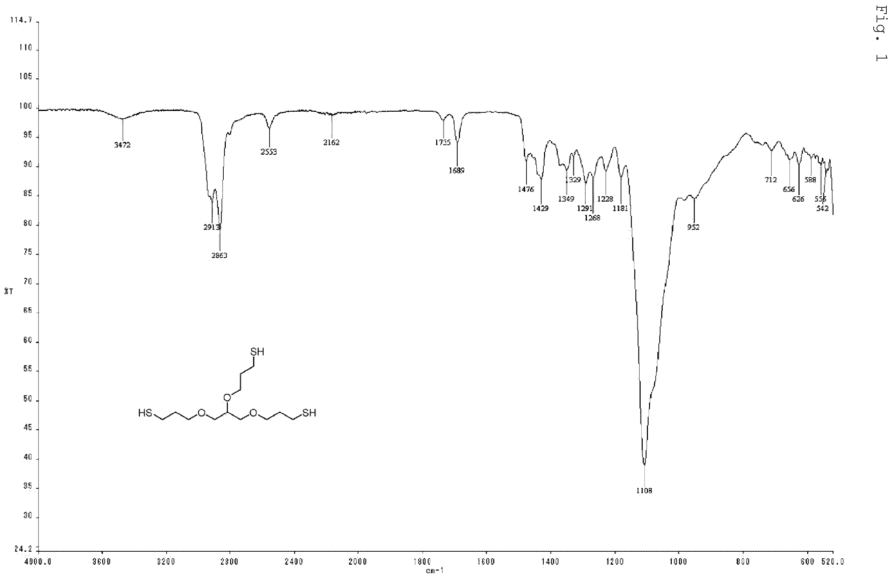

Fig. 1 is an IR spectrum chart of a colorless transparent liquid obtained in Synthesis Example 1.

### Description of Embodiments

### Thiol Compound

The curing agent of the present invention contains a thiol compound represented by formula (I) (1,2,3-tris(3-mercaptopropoxy)propane).

The thiol compound represented by formula (I) can be synthesized, for example, according to the following reaction formula (reaction scheme (A)). 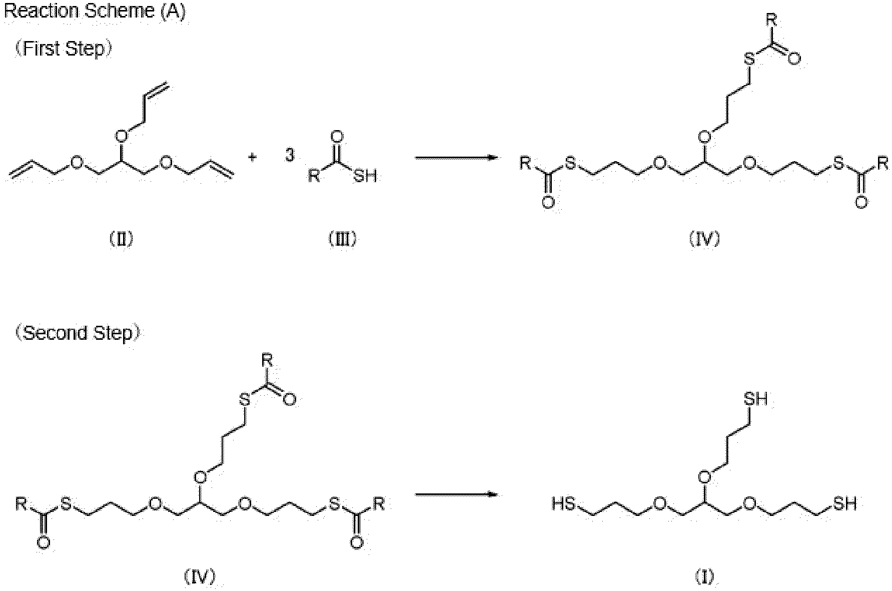 (In the formula, Rs may be the same or different and represent a methyl group, an ethyl group, a propyl group, or a phenyl group.)

### First Step

The first step is a step of reacting 1,2,3-triallyloxypropane represented by formula (II) with a thiocarboxylic acid represented by formula (III) to synthesize a compound represented by formula (IV).

1,2,3-triallyloxypropane represented by formula (II) can be synthesized, for example, according to the method described in JP2012-184198A.

From the viewpoint of reducing environmental impact, it is preferable to use 1,2,3-triallyloxypropane synthesized from raw materials derived from biomass. Specifically, 1,2,3-triallyloxypropane can be synthesized by using biomass-derived glycerin and biomass-derived allyl chloride as raw materials.

Examples of thiocarboxylic acids represented by formula (III) include thioacetic acid, thiobutyric acid, and thiobenzoic acid. These thiocarboxylic acids for use may be commercially available reagents.

In the first step, the amount (charged amount) of the thiocarboxylic acid represented by formula (III) to be used is preferably an appropriate proportion within the range of 1 to 10 times the molar amount of the allyl group of the 1,2,3-triallyloxypropane represented by formula (II).

In the first step, a radical initiator (a) may be used to promote the reaction. A reaction solvent (b) may also be used to facilitate the reaction.

Examples of the radical initiator (a) include azobisisobutyronitrile, t-hexylperoxyisopropyl monocarbonate, t-hexylperoxy 2-ethylhexanoate, 1,1,3,3-tetramethylbutylperoxy 2-ethylhexanoate, t-butylperoxypivalate, t-hexylperoxypivalate, t-butylperoxyneodecanoate, t-hexylperoxyneodecanoate, 1,1,3,3-tetramethylbutylperoxyneodecanoate, 1,1-bis(t-hexylperoxy)cyclohexane, benzoyl peroxide, 3,5,5-trimethylhexanoyl peroxide, lauroyl peroxide, 2,2'-azobis(2-methylbutyronitrile), and dimethyl 2,2'-azobis(2-methylpropionate).

The amount (charged amount) of the radical initiator (a) to be used is preferably an appropriate proportion within the range of 0.0001 to 10 times the molar amount of 1,2,3-triallyloxypropane represented by formula (II) to be used (charged amount).

Examples of the reaction solvent (b) include solvents such as water, methanol, ethanol, propanol, 2-propanol, butanol, ethyl acetate, propyl acetate, butyl acetate, tetrahydrofuran, dioxane, acetonitrile, benzene, toluene, xylene, dichloromethane, chloroform, carbon tetrachloride, dimethylformamide, dimethylacetamide, dimethylsulfoxide, and hexamethylphosphoric triamide. The reaction solvent (b) for use may be one of these or a combination of two or more.

In the first step, the reaction temperature is preferably set within the range of 0 to 150°C. The reaction time is set as appropriate according to the set reaction temperature, and is preferably set within the range of 1 to 120 hours.

After completion of the reaction in the first step, the reaction solvent and some other components may be distilled off from the resulting reaction solution (reaction mixture), and the reaction product obtained as a residue may be subjected to the second step. Alternatively, after completion of the reaction in the first step, the resulting reaction solution may be directly subjected to the second step.

### Second Step

The second step is a step of performing solvolysis (e.g., hydrolysis or alcoholysis) of the compound represented by formula (IV) to synthesize 1,2,3-tris(3-mercaptopropoxy)propane represented by formula (I).

Examples of alcohols used in the alcoholysis in the second step include methanol, ethanol, propanol, 2-propanol, butanol, ethylene glycol, propylene glycol, butylene glycol, and glycerin.

In the second step, it is preferable to use an acid (c) or a base (d) to promote the reaction. A reaction solvent (e) may also be used to facilitate the reaction.

Examples of the acid (c) include hydrogen fluoride, hydrogen chloride, hydrogen bromide, hydrogen iodide, carbonic acid, formic acid, acetic acid, benzoic acid, oxalic acid, citric acid, phosphoric acid, hexafluorophosphoric acid, nitric acid, sulfuric acid, methanesulfonic acid, toluenesulfonic acid, trifluoromethanesulfonic acid, boric acid, boron trifluoride, and tetrafluoroboric acid.

The amount (charged amount) of the acid (c) to be used is preferably an appropriate proportion within the range of 0.0001 to 10 times the molar amount of the used reaction product (produced amount) of the first step.

Examples of the base (d) include ammonia, trimethylamine, triethylamine, diisopropylethylamine, diazabicycloundecene, diazabicyclononene, pyridine, lithium hydroxide, sodium hydroxide, potassium hydroxide, cesium hydroxide, lithium carbonate, sodium carbonate, potassium carbonate, cesium carbonate, lithium hydrogen carbonate, sodium hydrogen carbonate, potassium hydrogen carbonate, cesium hydrogen carbonate, trilithium phosphate, trisodium phosphate, tripotassium phosphate, tricesium phosphate, dilithium hydrogen phosphate, disodium hydrogen phosphate, dipotassium hydrogen phosphate, dicesium hydrogen phosphate, lithium dihydrogen phosphate, sodium dihydrogen phosphate, potassium dihydrogen phosphate, cesium dihydrogen phosphate, lithium acetate, sodium acetate, potassium acetate, cesium acetate, sodium methoxide, sodium ethoxide, and potassium t-butoxide.

The amount (charged amount) of the base (d) to be used is preferably an appropriate proportion within the range of 2 to 200 times the molar amount of the used reaction product (produced amount) of the first step.

Examples of the reaction solvent (e) include ethyl acetate, propyl acetate, butyl acetate, tetrahydrofuran, dioxane, acetonitrile, benzene, toluene, xylene, dichloromethane, chloroform, carbon tetrachloride, dimethylformamide, dimethylacetamide, dimethylsulfoxide, and hexamethylphosphoric triamide. The reaction solvent (e) for use may be one of these or a combination of two or more.

In the second step, the reaction temperature is preferably set within the range of 0 to 150°C. The reaction time is set as appropriate according to the predetermined reaction temperature and is preferably set within the range of 1 to 120 hours.

After completion of the solvolytic reaction, the compound represented by formula (I) can be taken out from the obtained reaction solution according to a means such as concentration of the reaction solution by distillation of the reaction solvent or solvent extraction.

Additionally, the compound represented by formula (I) can be purified according to a means such as washing with water etc., activated carbon treatment, and silica gel chromatography, as necessary.

### Curing Agent

The compound represented by formula (I) can be used as a curing agent for resin, and the curing agent of the present invention contains the compound represented by formula (I).

The curing agent of the present invention may also contain, in addition to the compound represented by formula (I), a multimer of the compound represented by formula (I) (e.g., the compounds represented by formulas (I-1) to (I-4)) and/or an isomer (the compounds represented by formulas (I-5) to (I-9)).

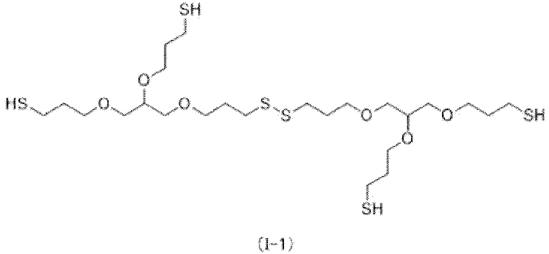

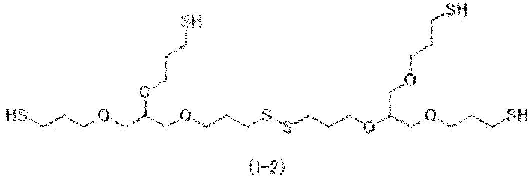

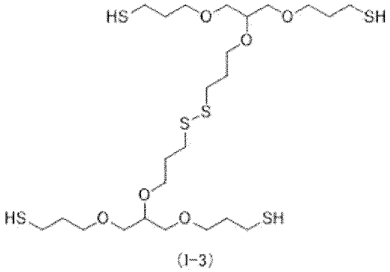

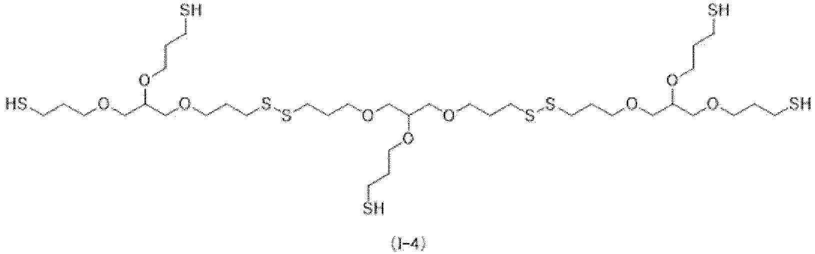

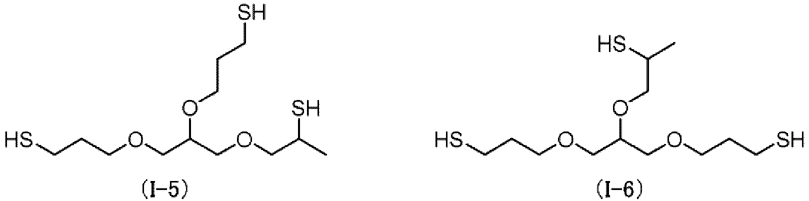

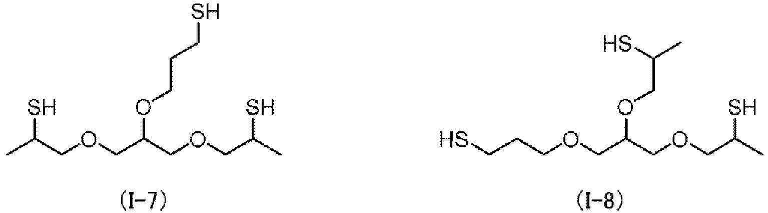

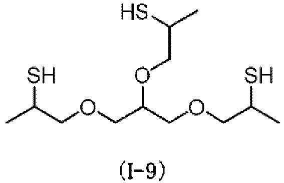

When the curing agent contains a multimer of the compound represented by formula (I), the ratio of the content of the multimer of the compound represented by formula (I) to the content of the compound represented by formula (I) in the curing agent is preferably 0.02 to 0.3, more preferably 0.05 to 0.25, and even more preferably 0.1 to 0.25.

When the curing agent contains an isomer of the compound represented by formula (I), the ratio of the content of the isomer of the compound represented by formula (I) to the content of the compound represented by formula (I) in the curing agent is preferably 0.01 to 0.3, more preferably 0.05 to 0.25, and even more preferably 0.07 to 0.2.

The ratio of the components contained in the curing agent is a value calculated using the size of the peak area of each component determined in the analysis of the curing agent by liquid chromatography.

### First Resin Composition

The first resin composition of the present invention is obtained by adding the curing agent of the present invention to an epoxy compound (which refers to an epoxy resin before curing).

Specifically, the first resin composition contains the curing agent of the present invention and an epoxy compound, and may further optionally contain other components such as a curing accelerator and a stabilizer.

The epoxy compound for use can be any compound that has an epoxy group (glycidyl group) in the molecule, without any particular limitation. Examples of epoxy compounds include diepoxy resins, such as polyethylene glycol diglycidyl ether, polypropylene glycol diglycidyl ether, butanediol diglycidyl ether, neopentyl glycol diglycidyl ether, 1,6-hexanediol diglycidyl ether, trimethylolpropane diglycidyl ether, polytetramethylene ether glycol diglycidyl ether, glycerin diglycidyl ether, cyclohexane-based diglycidyl ether, and dicyclopentadiene-based diglycidyl ether; triepoxy resins, such as trimethylolpropane triglycidyl ether and glycerin triglycidyl ether; polyglycidyl ethers (e.g., bisphenol-A epoxy resin and bisphenol-F epoxy resin) obtained by reacting polyphenols (e.g., bisphenol A, bisphenol F, bisphenol AD, catechol, and resorcinol) or polyhydric alcohols (e.g., glycerin and polyethylene glycol) with epichlorohydrin; glycidyl ether esters obtained by reacting hydroxycarboxylic acids, such as p-hydroxybenzoic acid and β-hydroxynaphthoic acid, with epichlorohydrin; polyglycidyl esters obtained by reacting polycarboxylic acids, such as phthalic acid and terephthalic acid, with epichlorohydrin; glycidyl glycoluril compounds having two or more epoxy groups in the molecule, such as 1,3,4,6-tetraglycidyl glycoluril; cycloaliphatic epoxy resins, such as 3',4'-epoxycyclohexylmethyl-3,4-epoxycyclohexanecarboxylate, vinyl (3,4-cyclohexene) dioxide, and 2-(3,4-epoxycyclohexyl)-5,1-spiro-(3,4-epoxycyclohexyl)-m-dioxane; dicyclopentadiene-based diglycidyl ethers, such as dicyclopentadiene dimethanol diglycidyl ether; cyclohexane-based diglycidyl ethers, such as 1,4-cyclohexanedimethanol diglycidyl ether; glycidylamine-based epoxy resins, such as tetraglycidyl bis(aminomethyl)cyclohexane; liquid epoxy resins with a naphthalene skeleton, such as 1,6-bis(glycidyloxy)naphthalene; epoxy resins with a silicone skeleton, such as 1,3-bis(3-glycidoxypropyl)-1,1,3,3-tetramethyldisiloxane; nitrogen-containing cyclic epoxy resins, such as triglycidyl isocyanurate and hydantoin-based epoxy resins (e.g., 1,3-diglycidyl-5-methyl-5-ethylhydantoin); epoxidized phenol novolac resins (phenol novolac-type epoxy resins), epoxidized cresol novolac resins, epoxidized polyolefins, cycloaliphatic epoxy resins, urethane-modified epoxy resins, and epoxy-modified organopolysiloxane compounds obtained by hydrosilylation addition reaction between an organic compound having a carbon-carbon double bond and a glycidyl group and a silicon compound having a SiH group (e.g., the epoxy-modified organopolysiloxane compounds disclosed in JP 2004-99751A and JP2006-282988A). These compounds may be used in combination.

The content of the curing agent of the present invention in the first resin composition of the present invention is preferably 0.1 to 70% by weight, more preferably 1 to 50% by weight, and even more preferably 20 to 40% by weight, based on the entire first resin composition (total amount).

The content of the curing agent of the present invention in the first resin composition is preferably set so that the ratio (equivalent ratio) of the number of thiol groups in the curing agent of the present invention to the number of epoxy groups in the composition is 0.1 to 10, and more preferably 0.5 to 1.5.

The first resin composition of the present invention may contain an additional thiol compound as a curing agent together with the curing agent of the present invention. Examples of such additional thiol compounds include aliphatic thiol compounds, such as ethanedithiol, propanedithiol, hexamethylenedithiol, decamethylenedithiol, tolylene-2,4-dithiol, 2,2-bis(mercaptomethyl)-1,3-propanedithiol, 2-(mercaptomethyl)-2-methyl-1,3-propanedithiol, and 2-ethyl-2-(mercaptomethyl)-1,3-propanedithiol; aromatic thiol compounds, such as benzenedithiol, toluenedithiol, and xylenedithiol (p-xylenedithiol); cyclic sulfide compounds, such as 1,4-dithiane ring-containing polythiol compounds represented by formula (V); mercaptoalkyl sulfide compounds, such as 3-thiapentane-1,5-dithiol and 4-mercaptomethyl-3,6-dithia-1,8-octanedithiol; mercaptopropionic esters, such as pentaerythritol tetrakis(3-mercaptopropionate); epoxy resin-terminated mercapto compounds; mercaptoalkyl ether compounds, such as 3,6-dioxa-1,8-octanedithiol, mercaptoalkyl ether disulfide compounds represented by formula (VI), 2,2'-[[2,2-bis[(2-mercaptoethoxy)methyl]-1,3-propanediyl]bis(oxy)]bisethanethiol, 3,3'-[[2,2-bis[(3-mercaptopropoxy)methyl]-1,3-propanediyl]bis(oxy)]bis-1-propanethiol, 3-[2,2-bis[(3-mercaptopropoxy)methyl]butoxy]-1-propanethiol, 3-(3-mercaptopropoxy)-2,2-bis[(3-mercaptopropoxy)methyl]-1-propanol, and 2,2-bis[(3-mercaptopropoxy)methyl]-1-butanol; and 1,3,4,6-tetrakis(2-mercaptoethyl)glycoluril and 1,3,4,6-tetrakis(3-mercaptopropyl)glycoluril, with 1,3,4,6-tetrakis(2-mercaptoethyl)glycoluril and 1,3,4,6-tetrakis(3-mercaptopropyl)glycoluril being preferred.

These additional thiol compounds may be used alone or in a combination of two or more. 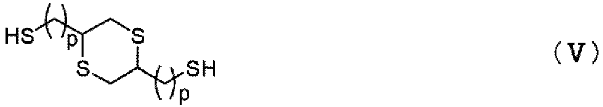 (In the formula, each p represents an integer of 1 to 5.) 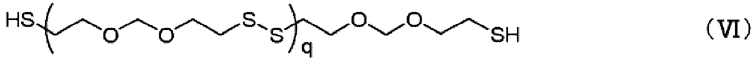 (In the formula, q represents an integer of 1 to 20.)

The content of the additional thiol compound in the first resin composition of the present invention is preferably set so that the ratio (equivalent ratio) of the number of thiol groups derived from the additional thiol compound in the composition to the number of thiol groups derived from the curing agent of the present invention is 0 to 100.

The first resin composition of the present invention may contain a known curing agent together with the curing agent of the present invention. Examples of such known curing agents include compounds having a phenolic hydroxy group, acid anhydrides, organic phosphine compounds, such as triphenylphosphine, diphenylnaphthylphosphine, and diphenylethylphosphine, aromatic phosphonium salts, aromatic diazonium salts, aromatic iodonium salts, and aromatic selenium salts.

Examples of compounds having a phenolic hydroxy group include bisphenol A, bisphenol F, bisphenol S, tetramethyl bisphenol A, tetramethyl bisphenol F, tetramethyl bisphenol S, tetrachlorobisphenol A, tetrabromobisphenol A, dihydroxynaphthalene, phenol novolac, cresol novolac, bisphenol A novolac, brominated phenol novolac, and resorcinol.

Examples of acid anhydrides include methyltetrahydrophthalic anhydride, methylhexahydrophthalic anhydride, hexahydrophthalic anhydride, 5-norbornene-2,3-dicarboxylic anhydride, trimellitic anhydride, nadic anhydride, himic anhydride, methylnadic anhydride, methylbicyclo[2.2.1]heptane-2,3-dicarboxylic anhydride, bicyclo[2.2.1]heptane-2,3-dicarboxylic anhydride, and methylnorbornane-2,3-dicarboxylic acid.

The first resin composition of the present invention may contain a known curing accelerator. Examples of curing accelerators include (i) amines, (ii) reaction products of an epoxy compound and an amine, and (iii) reaction products of a compound having one or more isocyanate groups in the molecule and a compound having either a primary amino group or a secondary amino group, or both, in the molecule. These curing accelerators may be used in combination.

As has been known, the amines (i) may be any amine having at least one amino group selected from the group consisting of a primary amino group, a secondary amino group, and a tertiary amino group in the molecule.

Examples of such amines include aliphatic amines, such as diethylenetriamine, triethylenetetramine, n-propylamine, 2-hydroxyethylaminopropylamine, cyclohexylamine, 4,4'-diaminodicyclohexylmethane, and dimethylbenzylamine; aromatic amines, such as 4,4'-diaminodiphenylmethane and o-methylaniline; and nitrogen-containing heterocyclic compounds, such as 2-ethyl-4-methylimidazole, 2-methylimidazole, 2-ethyl-4-methylimidazoline, 2,4-dimethylimidazoline, piperidine, and piperazine.

The content of the curing accelerator (in particular, amines) in the first resin composition of the present invention is preferably 0.1 to 30 parts by weight, and more preferably 1 to 10 parts by weight, per 100 parts by weight of the curing agent of the present invention.

The reaction product of an epoxy compound and an amine (ii) is a solid that is poorly soluble in an epoxy resin at room temperature, but is solubilized (become easily soluble) by heating and functions as a curing accelerator; thus, the reaction product of an epoxy compound and an amine (ii) is also called a "latent curing accelerator." (the reaction product of an epoxy compound and an amine may be referred to below as a "latent curing accelerator.")

Examples of epoxy compounds for use as a raw material for the latent curing accelerator include, in addition to the epoxy compounds described above, glycidylamine compounds obtained by reacting 4,4'-diaminodiphenylmethane, m-aminophenol, or the like with epichlorohydrin; and monofunctional epoxy compounds, such as butyl glycidyl ether, phenyl glycidyl ether, and glycidyl methacrylate.

Examples of amines for use as a raw material for the latent curing accelerator include the amines described above. Of these amines, those having a tertiary amino group in the molecule serve as raw materials that provide latent curing accelerators having excellent curing acceleration properties. Examples of amines include amines such as dimethylaminopropylamine, diethylaminopropylamine, di-n-propylaminopropylamine, dibutylaminopropylamine, dimethylaminoethylamine, diethylaminoethylamine, and N-methylpiperazine; amines having a tertiary amino group in the molecule, such as imidazole compounds, including 2-methylimidazole, 2-ethylimidazole, 2-ethyl-4-methylimidazole, and 2-phenylimidazole; and alcohols, phenols, thiols, carboxylic acids, and hydrazides having a tertiary amino group in the molecule, such as 2-dimethylaminoethanol, 1-methyl-2-dimethylaminoethanol, 1-phenoxymethyl-2-dimethylaminoethanol, 2-diethylaminoethanol, 1-butoxymethyl-2-dimethylaminoethanol, 1-(2-hydroxy-3-phenoxypropyl)-2-methylimidazole, 1-(2-hydroxy-3-phenoxypropyl)-2-ethyl-4-methylimidazole, 1-(2-hydroxy-3-butoxypropyl)-2-methylimidazole, 1-(2-hydroxy-3-butoxypropyl)-2-ethyl-4-methylimidazole, 1-(2-hydroxy-3-phenoxypropyl)-2-phenylimidazoline, 1-(2-hydroxy-3-butoxypropyl)-2-methylimidazoline, 2-(dimethylaminomethyl)phenol, 2,4,6-tris(dimethylaminomethyl)phenol, N-β-hydroxyethylmorpholine, 2-dimethylaminoethanethiol, 2-mercaptopyridine, 2-mercaptobenzimidazole, 2-mercaptobenzothiazole, 4-mercaptopyridine, N,N-dimethylaminobenzoic acid, N,N-dimethylglycine, nicotinic acid, isonicotinic acid, picolinic acid, N,N-dimethylglycine hydrazide, N,N-dimethylpropionic acid hydrazide, nicotinic acid hydrazide, and isonicotinic acid hydrazide.

To further improve the storage stability of the first resin composition of the present invention, an active hydrogen compound having two or more active hydrogens in the molecule may be used as a third component in addition to the epoxy compound and amine described above, as a raw material for the latent curing accelerator.

Examples of active hydrogen compounds include polyhydric phenols, such as bisphenol A, bisphenol F, bisphenol S, hydroquinone, catechol, resorcinol, pyrogallol, and phenol novolac resin; polyhydric alcohols, such as trimethylolpropane; polyvalent carboxylic acids, such as adipic acid and phthalic acid; 1,2-dimercaptoethane, 2-mercaptoethanol, 1-mercapto-3-phenoxy-2-propanol, mercaptoacetic acid, anthranilic acid, and lactic acid.

Additionally, the latent curing accelerator may be surface-treated with an isocyanate compound or an acidic compound. Examples of isocyanate compounds include monofunctional isocyanate compounds, such as n-butyl isocyanate, isopropyl isocyanate, phenyl isocyanate, and benzyl isocyanate; and polyfunctional isocyanate compounds, such as hexamethylene diisocyanate, toluylene diisocyanate, 1,5-naphthalene diisocyanate, diphenylmethane-4,4'-diisocyanate, isophorone diisocyanate, xylylene diisocyanate, paraphenylene diisocyanate, 1,3,6-hexamethylene triisocyanate, and bicycloheptane triisocyanate.

Instead of the polyfunctional isocyanate compounds, isocyanate-terminated compounds obtained by reacting a polyfunctional isocyanate compound with an active hydrogen compound are also usable. Specific examples include isocyanate-terminated addition reaction products obtained by reacting toluylene diisocyanate with trimethylolpropane, and isocyanate-terminated addition reaction products obtained by reacting toluylene diisocyanate with pentaerythritol.

The acidic substance for use in the surface treatment of the latent curing accelerator may be in any form of gas, liquid, or solid, and may be either an inorganic acid or an organic acid. Examples of acidic substances include carbon dioxide gas, sulfur dioxide gas, sulfuric acid, hydrochloric acid, oxalic acid, phosphoric acid, acetic acid, formic acid, propionic acid, adipic acid, caproic acid, lactic acid, succinic acid, tartaric acid, sebacic acid, p-toluenesulfonic acid, salicylic acid, boric acid, tannic acid, alginic acid, polyacrylic acid, polymethacrylic acid, phenol, pyrogallol, phenolic resin, and resorcinol resin.

The latent curing accelerator can be easily obtained by mixing an epoxy compound and an amine, optionally with an active hydrogen compound, reacting the mixture at a temperature within the range of room temperature to 200°C, solidifying and pulverizing the mixture, or by reacting the mixture in a solvent such as methyl ethyl ketone, dioxane, or tetrahydrofuran, removing the solvent, and then pulverizing the solid.

Alternatively, a commercially available latent curing accelerator may be used. Examples of commercially available products include Amicure PN-23 (trade name), Amicure PN-H (trade name), Amicure PN-50 (trade name), Amicure PN-23J (trade name), Amicure PN-40J (trade name), and Amicure MY-24 (trade name) manufactured by Ajinomoto Fine-Techno Co., Inc.; Novacure HX-3088 (trade name), Novacure HX-3721 (trade name), Novacure HX-3722 (trade name), Novacure HX-3742 (trade name), Novacure HX-3941HP (trade name), and Novacure HXA3922HP (trade name) manufactured by Asahi Kasei Corporation; and Fujicure FXR-1030 (trade name), Fujicure FXR-1081 (trade name), and Fujicure FXR-1121 (trade name) manufactured by T&K TOKA Corporation.

The content of the latent curing accelerator in the first resin composition of the present invention is preferably 0.1 to 1000 parts by weight, more preferably 1 to 80 parts by weight, and even more preferably 1 to 30 parts by weight, per 100 parts by weight of the epoxy compound (epoxy resin).

The reaction products of a compound having one or more isocyanate groups in the molecule and a compound having either a primary amino group or a secondary amino group, or both, in the molecule (iii) can be obtained by reacting both compounds in an organic solvent such as dichloromethane.

Examples of isocyanate compounds having one or more isocyanate groups in the molecule include n-butyl isocyanate, isopropyl isocyanate, 2-chloroethyl isocyanate, phenyl isocyanate, p-bromophenyl isocyanate, m-chlorophenyl isocyanate, o-chlorophenyl isocyanate, p-chlorophenyl isocyanate, 2,5-dichlorophenyl isocyanate, 3,4-dichlorophenyl isocyanate, 2,6-dimethylphenyl isocyanate, o-fluorophenyl isocyanate, p-fluorophenyl isocyanate, m-tolyl isocyanate, p-tolyl isocyanate, o-trifluoromethylphenyl isocyanate, m-trifluoromethylphenyl isocyanate, benzyl isocyanate, hexamethylene diisocyanate, 2,4-toluylene diisocyanate, 2,6-toluylene diisocyanate, 1,5-naphthalene diisocyanate, diphenylmethane-4,4'-diisocyanate, 2,2-dimethyldiphenylmethane-4,4'-diisocyanate, tolidine diisocyanate, isophorone diisocyanate, xylylene diisocyanate, 1,3-bis(isocyanatomethyl)cyclohexane, p-phenylene diisocyanate, 1,3,6-hexamethylene triisocyanate, bicycloheptane triisocyanate, tris-(3-isocyanato-4-methylphenyl)isocyanurate, and tris-(6-isocyanatohexyl)isocyanurate.

Examples of compounds having either a primary amino group or a secondary amino group, or both, in the molecule include dimethylamine, diethylamine, di-n-propylamine, di-n-butylamine, di-n-hexylamine, di-n-octylamine, di-n-ethanolamine, dimethylaminopropylamine, diethylaminopropylamine, morpholine, piperidine, 2,6-dimethylpiperidine, 2,2,6,6-tetramethylpiperidine, piperazine, pyrrolidine, benzylamine, N-methylbenzylamine, cyclohexylamine, metaxylylenediamine, 1,3-bis(aminomethyl)cyclohexane, isophoronediamine, N-aminoethylpiperazine, 2-methylimidazole, 2-ethyl-4-methylimidazole, 2-undecylimidazole, 2-phenylimidazole, and 1,1-dimethylhydrazine.

In the first resin composition of the present invention, the content of the reaction product of a compound having one or more isocyanate groups in the molecule and a compound having either a primary amino group or a secondary amino group, or both, in the molecule is preferably 1 to 10 parts by weight per 100 parts by weight of the epoxy compound (epoxy resin).

The first resin composition of the present invention may contain a known stabilizer as long as the stabilizer does not impair the effects of the present invention. Examples of stabilizers include liquid boric ester compounds, aluminum chelates (e.g., aluminum trisacetylacetonate), and organic acids (e.g., acetic acid, propionic acid, butyric acid, succinic acid, malic acid, citric acid, and barbituric acid).

Examples of liquid boric ester compounds include trimethyl borate, triethyl borate, tri-n-propyl borate, triisopropyl borate, triallyl borate, tri-n-butyl borate, tripentyl borate, trihexyl borate, tricyclohexyl borate, trioctyl borate, trinonyl borate, tridecyl borate, tridodecyl borate, trihexadecyl borate, trioctadecyl borate, tris(2-ethylhexyloxy)borane, triphenyl borate, tri-o-tolyl borate, trim-tolyl borate, tribenzyl borate, triethanolamine borate, and 2,2'-oxybis(5,5'-dimethyl-1,3,2-oxaborinane).

The liquid boric ester compounds are in a liquid form at room temperature and thus can limit an increase in viscosity of resin compositions.

The content of the stabilizer in the first resin composition of the present invention is preferably 0.1 to 9% by weight, more preferably 0.1 to 5% by weight, and even more preferably 0.1 to 4% by weight based on the entire first resin composition (total amount).

The first resin composition of the present invention may contain triphenylsilanol. Due to triphenylsilanol contained, the first resin composition of the present invention can maintain its pot life and can have a lower curing termination temperature when being cured.

The content of triphenylsilanol in the first resin composition of the present invention is preferably 0.1 to 10% by weight, more preferably 0.3 to 7% by weight, and even more preferably 0.5 to 6% by weight, based on the entire first resin composition (total amount).

The first resin composition of the present invention may contain talc. Due to talc contained, the resin composition of the present invention can have increased heat resistance, low thermal expansion properties, and impact resistance.

The talc preferably has a plate-like or flat shape.

The talc preferably has an aspect ratio of 5 to 20.

The content of talc in the first resin composition of the present invention is preferably 5 to 20 parts by weight, and more preferably 5 to 15 parts by weight, per 100 parts by weight of the epoxy compound (epoxy resin).

The first resin composition of the present invention may contain a filler. Due to a filler contained, the cured product of the first resin composition can have a decreased linear expansion coefficient, resulting in improved moisture resistance and thermal cycle resistance.

Examples of fillers include silica filler (e.g., fused silica and spherical silica), alumina filler (e.g., spherical alumina and crushed alumina), kaolin, clay, mica, barium sulfate, lithopone, gypsum, zinc stearate, oxides such as perlite, quartz, quartz glass, magnesium oxide, beryllium oxide, and titanium oxide, nitrides such as boron nitride, silicon nitride, and aluminum nitride, carbides such as silicon carbide, hydroxides such as aluminum hydroxide and magnesium hydroxide, metals and alloys such as copper, silver, iron, aluminum, nickel, and titanium, and carbon-based materials such as diamond and carbon.

The filler preferably has an average particle size of 0.005 to 10 µm, and more preferably 0.1 to 6 µm.

The filler may have a shape such as a spherical, irregular, or scaly shape. For the filler with a shape other than a spherical shape, the average particle size of the filler means the average maximum diameter of the filler.

The content of the filler in the first resin composition of the present invention is preferably 0 to 400 parts by weight, more preferably 5 to 300 parts by weight, and even more preferably 5 to 200 parts by weight, per 100 parts by weight of the entire first resin composition excluding the filler (total amount).

The first resin composition of the present invention may contain calcium carbonate. Due to calcium carbonate contained, the cured product of the first resin composition can have increased drop impact resistance.

The average particle size of calcium carbonate is not particularly limited, but is preferably 0.1 to 15 µm.

The content of calcium carbonate in the first resin composition of the present invention is preferably 5 to 200 parts by weight per 100 parts by weight of the epoxy compound (epoxy resin).

The first resin composition of the present invention may contain a reactive diluent. In the present specification, the reactive diluent refers to a compound that has one epoxy group (glycidyl group) and that has a relatively low viscosity at room temperature.

The reactive diluent may have, in addition to the epoxy group, other polymerizable functional groups, such as alkenyl groups (e.g., vinyl and allyl) and unsaturated carboxylic acid residues (e.g., acryloyl and methacryloyl).

Examples of reactive diluents include monoepoxide compounds, such as n-butyl glycidyl ether, 2-ethylhexyl glycidyl ether, phenyl glycidyl ether, cresyl glycidyl ether, p-s-butylphenyl glycidyl ether, styrene oxide, and α-pinene oxide; and monoepoxide compounds having other functional groups, such as allyl glycidyl ether, glycidyl methacrylate, and 1-vinyl-3,4-epoxycyclohexane.

The content of the reactive diluent in the first resin composition of the present invention is preferably 1 to 150 parts by weight per 100 parts by weight of the epoxy compound (epoxy resin).

The first resin composition of the present invention may optionally contain the following in an amount of 0.01 to 50% by weight based on the entire first resin composition (total amount), as long as the effects of the present invention are not impaired: pigments (e.g., titanium white, cyanine blue, watching red, red iron oxide, carbon black, aniline black, manganese blue, iron black, ultramarine blue, Hansa red, chrome yellow, and chrome green); thermoplastic resins and/or thermosetting resins (e.g., homopolymers such as high-density, medium-density, and low-density polyethylenes, polypropylene, polybutene, and polypentene, ethylene-propylene copolymers, polyamide resins such as nylon-6 and nylon-6,6, vinyl chloride resins, nitrocellulose resins, vinylidene chloride resins, acrylic resins, acrylamide resins, styrene resins, vinyl ester resins, polyester resins, phenol resins (phenolic compounds), silicone resins, fluorine resins, elastomer resins such as acrylic rubber and urethane rubber, graft copolymers such as methyl methacrylate-butadiene-styrene graft copolymers and acrylonitrile-butadiene-styrene graft copolymers); reinforcing agents (e.g., glass fibers and carbon fibers); anti-sagging agents (e.g., hydrogenated castor oil and fine silicic anhydride); matting agents (e.g., fine silica and paraffin wax); grinding agents (e.g., zinc stearate); internal mold release agents (e.g., fatty acids such as stearic acid, fatty acid metal salts of calcium stearate, fatty acid amides such as stearic acid amide, fatty acid esters, polyolefin wax, and paraffin wax); additives (modifiers), such as surfactants, leveling agents, defoamers, viscosity-adjusting diluents (organic solvents), flexibility-imparting agents, coupling agents (e.g., silane coupling agents such as glycidyl silane coupling agents and titanium coupling agents), fragrances, flame retardants, and antioxidants.

Additionally, adding an isocyanate group-containing compound as an additive (modifier) to the first resin composition of the present invention can increase adhesive power while limiting the decrease in curability of the resin composition.

Examples of isocyanate group-containing compounds include n-butyl isocyanate, isopropyl isocyanate, 2-chloroethyl isocyanate, phenyl isocyanate, p-chlorophenyl isocyanate, benzyl isocyanate, hexamethylene diisocyanate, 2-ethylphenyl isocyanate, 2,6-dimethylphenyl isocyanate, 2,4-toluene diisocyanate, 2,6-toluene diisocyanate, 1,5-naphthalene diisocyanate, diphenylmethane-4,4'-diisocyanate, tolidine diisocyanate, isophorone diisocyanate, xylylene diisocyanate, paraphenylene diisocyanate, 1,3,6-hexamethylene triisocyanate, and bicycloheptane triisocyanate.

The content of the isocyanate group-containing compound in the first resin composition of the present invention is preferably 0.1 to 20 parts by weight per 100 parts by weight of the epoxy compound (epoxy resin).

The method for preparing (mixing) the first resin composition of the present invention is not particularly limited. The components described above can be taken in predetermined amounts and weighed and then mixed by using an appropriate stirring and mixing device such as a three-roll mill or a planetary mixer, optionally with heating.

The method for curing the first resin composition of the present invention is not particularly limited. A known curing device such as a sealed curing oven or a tunnel oven capable of continuous curing can be used. The heating source is also not particularly limited, and any known means such as hot air circulation, infrared heating, and high-frequency heating can be used. The curing temperature and curing time may be set as appropriate.

### Second Resin Composition

The second resin composition of the present invention contains the curing agent of the present invention and an ene compound having a carbon-carbon double bond in the molecule (which may be simply referred to as an "ene compound" below). Specifically, the second resin composition contains the curing agent of the present invention and an ene compound, and may further optionally contain a photopolymerization initiator, a stabilizer, etc.

The ene compound encompasses both a polymerizable monomer and a polymerizable oligomer having a structure in which a polymerizable monomer is partially polymerized (semi-cured product).

Examples of the polymerizable monomer include
(1) (meth)acrylic acid alkyl ester monomers,
(2) hydroxy group-containing monomers,
(3) carboxyl group-containing monomers,
(4) amino group-containing monomers,
(5) acetoacetyl group-containing monomers,
(6) isocyanate group-containing monomers,
(7) glycidyl group-containing monomers,
(8) monomers containing one aromatic ring,
(9) monomers containing an alkoxy group and an oxyalkylene group,
(10) alkoxyalkyl (meth)acrylamide monomers,
(11) (meth)acrylamide monomers,
(12) monofunctional unsaturated compounds, and
(13) polyfunctional unsaturated compounds.

(1) Examples of the (meth)acrylic acid alkyl ester monomers include methyl (meth)acrylate, ethyl (meth)acrylate, n-butyl (meth)acrylate, iso-butyl (meth)acrylate, tert-butyl (meth)acrylate, n-propyl (meth)acrylate, n-hexyl (meth)acrylate, 2-ethylhexyl (meth)acrylate, n-octyl (meth)acrylate, isodecyl (meth)acrylate, lauryl (meth)acrylate, cetyl (meth)acrylate, stearyl (meth)acrylate, cyclohexyl (meth)acrylate, and isobornyl (meth)acrylate.
(2) Examples of the hydroxy group-containing monomers include (meth)acrylic acid hydroxyalkyl esters, such as 2-hydroxyethyl (meth)acrylate, 4-hydroxybutyl (meth)acrylate, 5-hydroxypentyl (meth)acrylate, 6-hydroxyhexyl (meth)acrylate, and 8-hydroxyoctyl (meth)acrylate; caprolactone-modified monomers, such as caprolactone-modified 2-hydroxyethyl (meth)acrylate; oxyalkylene-modified monomers, such as diethylene glycol (meth)acrylate and polyethylene glycol (meth)acrylate; primary hydroxy group-containing monomers, such as 2-acryloyloxyethyl-2-hydroxyethyl phthalic acid, N-methylol (meth)acrylamide, and hydroxyethyl acrylamide; secondary hydroxy group-containing monomers, such as 2-hydroxypropyl (meth)acrylate, 2-hydroxybutyl (meth)acrylate, 3-chloro-2-hydroxypropyl (meth)acrylate, propylene glycol diglycidyl ether-epoxy di(meth)acrylate, phenol glycidyl ether-epoxy(meth)acrylate, and bisphenol A diglycidyl ether-epoxy di(meth)acrylate; and tertiary hydroxy group-containing monomers, such as 2,2-dimethyl 2-hydroxyethyl (meth)acrylate.
(3) Examples of the carboxyl group-containing monomers include (meth)acrylic acid, acrylic acid dimer, crotonic acid, maleic acid, maleic anhydride, fumaric acid, citraconic acid, glutaconic acid, itaconic acid, acrylamide-N-glycolic acid, and cinnamic acid.
(4) Examples of the amino group-containing monomers include tert-butylaminoethyl (meth)acrylate, ethylaminoethyl (meth)acrylate, dimethylaminoethyl (meth)acrylate, and diethylaminoethyl (meth)acrylate.
(5) Examples of the acetoacetyl group-containing monomers include 2-(acetoacetoxy)ethyl (meth)acrylate and allylacetoacetate.
(6) Examples of the isocyanate group-containing monomers include 2-acryloyloxyethyl isocyanate, 2-methacryloyloxyethyl isocyanate, and alkylene oxide adducts thereof.
(7) Examples of the glycidyl group-containing monomers include glycidyl (meth)acrylate; epoxy (meth)acrylates as reaction products of an epoxy compound and (meth)acrylic acid, such as ethylene glycol diglycidyl ether-epoxy (meth)acrylate, resorcin diglycidyl ether-epoxy (meth)acrylate, bis(4-hydroxyphenyl) sulfide diglycidyl ether-epoxy (meth)acrylate, phenolic novolac epoxy resin-(meth)acrylate, cresol novolac epoxy resin-(meth)acrylate, bisphenol (e.g., bisphenol A, bisphenol F) epoxy resin-(meth)acrylate, biphenol (e.g., 3,3',5,5'-tetramethylbiphenol) epoxy resin-(meth)acrylate, and tris(2,3-epoxypropyl) isocyanurate-(meth)acrylate; and glycidyl (meth)acrylates, such as 4-hydroxybutyl (meth)acrylate glycidyl ether.
(8) Examples of the monomers containing one aromatic ring include phenyl (meth)acrylate, benzyl (meth)acrylate, phenoxyethyl (meth)acrylate, phenoxydiethylene glycol (meth)acrylate, 2-hydroxy-3-phenoxypropyl (meth)acrylate, styrene, and α-methylstyrene.
(9) Examples of the monomers containing an alkoxy group and an oxyalkylene group include 2-methoxyethyl (meth)acrylate, 2-ethoxyethyl (meth)acrylate, 3-methoxybutyl (meth)acrylate, 2-butoxyethyl (meth)acrylate, 2-butoxydiethylene glycol (meth)acrylate, methoxydiethylene glycol (meth)acrylate, methoxytriethylene glycol (meth)acrylate, ethoxydiethylene glycol (meth)acrylate, methoxydipropylene glycol (meth)acrylate, methoxypolyethylene glycol (meth)acrylate, octoxypolyethylene glycol-polypropylene glycol-mono(meth)acrylate, lauroxypolyethylene glycol mono(meth)acrylate, and stearoxypolyethylene glycol mono(meth)acrylate.
(10) Examples of the alkoxyalkyl (meth)acrylamide monomers include methoxymethyl (meth)acrylamide, ethoxymethyl (meth)acrylamide, propoxymethyl (meth)acrylamide, isopropoxymethyl (meth)acrylamide, n-butoxymethyl (meth)acrylamide, and isobutoxymethyl (meth)acrylamide.
(11) Examples of the (meth)acrylamide monomers include (meth)acryloylmorpholine, dimethyl (meth)acrylamide, diethyl (meth)acrylamide, and (meth)acrylamide N-methylol (meth)acrylamide.
(12) Examples of the monofunctional unsaturated compounds include biphenyl structure-containing (meth)acrylate compounds. More specific examples include biphenyl (meth)acrylates, such as o-biphenyl (meth)acrylate, m-biphenyl (meth)acrylate, and p-biphenyl (meth)acrylate; biphenyloxyalkyl (meth)acrylates, such as o-biphenyloxymethyl (meth)acrylate, m-biphenyloxymethyl (meth)acrylate, p-biphenyloxymethyl (meth)acrylate, o-biphenyloxyethyl (meth)acrylate, m-biphenyloxyethyl (meth)acrylate, p-biphenyloxyethyl (meth)acrylate, o-biphenyloxypropyl (meth)acrylate, m-biphenyloxypropyl (meth)acrylate, and p-biphenyloxypropyl (meth)acrylate; and biphenyloxy polyalkylene glycol (meth)acrylates, such as (o-biphenyloxy)diethylene glycol (meth)acrylate, (m-biphenyloxy)diethylene glycol (meth)acrylate, (p-biphenyloxy)diethylene glycol (meth)acrylate, (o-biphenyloxy)dipropylene glycol (meth)acrylate, (m-biphenyloxy)dipropylene glycol (meth)acrylate, (p-biphenyloxy)dipropylene glycol (meth)acrylate, (o-biphenyloxy)polyethylene glycol (meth)acrylate, (m-biphenyloxy)polyethylene glycol (meth)acrylate, (p-biphenyloxy)polyethylene glycol (meth)acrylate, (o-biphenyloxy)polypropylene glycol (meth)acrylate, (m-biphenyloxy)polypropylene glycol (meth)acrylate, and (p-biphenyloxy)polypropylene glycol (meth)acrylate.
(13) Examples of the polyfunctional unsaturated compounds include bifunctional monomers, trifunctional or higher functional monomers, urethane (meth)acrylates, the epoxy (meth)acrylates mentioned above, polyester (meth)acrylates, and polyether (meth)acrylates.

Specific examples of bifunctional monomers include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, polypropylene glycol di(meth)acrylate, butylene glycol di(meth)acrylate, neopentyl glycol di(meth)acrylate, ethylene oxide-modified bisphenol A di(meth)acrylate, propylene oxide-modified bisphenol A di(meth)acrylate, 1,6-hexanediol di(meth)acrylate, 1,6-hexanediol ethylene oxide-modified di(meth)acrylate, glycerin di(meth)acrylate, pentaerythritol di(meth)acrylate, ethylene glycol diglycidyl ether di(meth)acrylate, diethylene glycol diglycidyl ether di(meth)acrylate, phthalic acid diglycidyl ester di(meth)acrylate, hydroxypivalic acid-modified neopentyl glycol di(meth)acrylate, isocyanuric acid ethylene oxide-modified diacrylate, and 2-(meth)acryloyloxyethyl acid phosphate diester.

Specific examples of trifunctional or higher functional monomers include trimethylolpropane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, dipentaerythritol hexa(meth)acrylate, tri(meth)acryloyloxy ethoxy trimethylolpropane, glycerin polyglycidyl ether poly(meth)acrylate, tris(2-(meth)acryloyloxyethyl)isocyanurate, isocyanuric acid ethylene oxide-modified tri(meth)acrylate, ethylene oxide-modified dipentaerythritol penta(meth)acrylate, ethylene oxide-modified dipentaerythritol hexa(meth)acrylate, ethylene oxide-modified pentaerythritol tri(meth)acrylate, ethylene oxide-modified pentaerythritol tetra(meth)acrylate, and succinic acid-modified pentaerythritol tri(meth)acrylate.

In addition to the polymerizable monomers above, examples also include divinylbenzene, piperylene, isoprene, pentadiene, vinylcyclohexene, chloroprene, butadiene, methylbutadiene, cyclopentadiene, methylpentadiene, acrylonitrile, methacrylonitrile, vinyl acetate, vinyl propionate, vinyl stearate, vinyl chloride, vinylidene chloride, alkyl vinyl ether, vinyl toluene, vinyl pyridine, vinyl pyrrolidone, dialkyl itaconate, dialkyl fumarate, allyl alcohol, acryloyl chloride, methyl vinyl ketone, N-acrylamidomethyltrimethylammonium chloride, allyltrimethylammonium chloride, dimethylallyl vinyl ketone, 2-chloroethyl vinyl ether, triallyl isocyanurate, tetraallyl glycoluril, N-vinylpyrrolidone, N-vinylcaprolactam, ethylene glycol diallyl carbonate, trimellitic acid triallyl ester, trifluoroethyl (meth)acrylate, tribromobenzyl (meth)acrylate, perfluorooctylethyl (meth)acrylate, sulfur-containing (meth)acrylate, and (meth)acryloyloxypropyl tris(methoxy)silane.

In the second resin composition of the present invention, the ene compound for use may be a combination of the polymerizable monomer and the polymerizable oligomer described above. The polymerizable monomer for use may be a combination of the polymerizable monomers described as examples above (which may be a combination of different types of polymerizable monomers), and the polymerizable oligomer for use may be a combination of different types of polymerizable oligomers.

In regards to the content ratio (proportion) of the curing agent of the present invention and the ene compound in the second resin composition of the present invention, the content of the ene compound is preferably an appropriate proportion within the range of 0.7 to 10 times (ratio by weight) the content of the curing agent of the present invention, and more preferably within the range of 1 to 5 times (ratio by weight) the content of the curing agent of the present invention.

In the second resin composition of the present invention, the additional thiol compound described above may be used in combination with the curing agent of the present invention.

In regards to the content ratio (proportion) of the curing agent of the present invention and the additional thiol compound in the second resin composition of the present invention, the content of the additional thiol compound is an appropriate proportion preferably within the range of 0 to 100 times (ratio by weight) the content of the curing agent of the present invention, and more preferably within the range of 0.1 to 10 times (ratio by weight) the content of the curing agent of the present invention.

The method for polymerizing (curing) the second resin composition of the present invention includes light curing and heat curing.

The light curing method includes a method of irradiation with active energy rays, and preferably a method that uses a photopolymerization initiator in combination. Active energy rays include light, radioactive rays, electromagnetic waves, electron beams, and the like. Preferred are electron beams or light in the ultraviolet to infrared wavelength range. For example, the light source for use for ultraviolet irradiation may be an ultra-high-pressure mercury light source or a metal halide light source, the light source for use for visible light irradiation may be a metal halide light source or a halogen light source, and the light source for use for infrared irradiation may be a halogen light source. Light sources that have been increasingly used recently, such as lasers and LEDs that emit light at various wavelengths, may also be used. The irradiation amount of active energy rays can be appropriately set, for example, according to the type of the light source.

The photopolymerization initiator can be selected from a photo-radical polymerization initiator and a photo-anionic polymerization initiator, and these initiators can be added to the second resin composition. In light curing, the means of thermal polymerization (heat curing) may be used in combination to improve production efficiency and the characteristics of the cured product.

The photo-radical polymerization initiator for use can be any commonly used photo-radical polymerization initiator, without any particular limitation. Examples of photo-radical polymerization initiators include acetophenones, such as 2-hydroxy-2-methyl-1-phenylpropan-1-one, 1-hydroxycyclohexyl phenyl ketone, and 2-methyl-1-{4-(methylthio)phenyl}-2-morpholinopropan-1-one; benzoins, such as benzyl dimethyl ketal; benzophenones, such as benzophenone, 4-phenylbenzophenone, and hydroxybenzophenone; thioxanthones, such as isopropylthioxanthone and 2,4-diethylthioxanthone; and methylphenyl glyoxylate. These may be used in combination.

The photo-radical polymerization initiator may be used optionally in combination with a known photopolymerization accelerator, such as a benzoic acid (e.g., 4-dimethylaminobenzoic acid) or a tertiary amine.

The photo-anionic polymerization initiator for use can be any commonly used photo-anionic polymerization initiator, without any particular limitation, and examples include onium salts and carbamates.

Examples of onium salts include 1,2-diisopropyl-3-(bis(dimethylamino)methylene)guanidium 2-(3-benzoylphenyl)propionate, and 1,2-dicyclohexyl-4,4,5,5-tetramethylbiguanidium n-butyltriphenylborate. Examples of carbamates include 2-nitrophenylmethylpiperidine-1-carboxylate, 1-(anthraquinon-2-yl)ethylimidazolecarboxylate, 1-(3-(2-hydroxyphenyl)-2-propenoyl)piperidine, and 9-anthranylmethyl diethyl carbamate.

To photo-cure the second resin composition of the present invention, a sensitizer, such as pyrene, perylene, acridine orange, thioxanthone, 2-chlorothioxanthone, or benzoflavin, can be used.

The content of the photopolymerization initiator in the second resin composition of the present invention is preferably 0.001 to 20% by weight, and more preferably 0.01 to 10% by weight, based on the entire second resin composition (total amount) .

The method for heat-curing the second resin composition of the present invention includes a method that uses a thermal polymerization initiator in combination. The thermal polymerization initiator can be selected from a thermal radical polymerization initiator and a thermal anionic polymerization initiator, and these initiators can be added to the resin composition.

For the conditions of heat curing, the heating temperature and heating time can be appropriately set. The heating temperature and heating time are preferably set within the range of 60 to 130°C and the range of 30 to 240 minutes, and more preferably within the range of 70 to 125°C and the range of 30 to 120 minutes.

The thermal radical polymerization initiator for use can be any commonly used thermal radical polymerization initiator, without any particular limitation. Examples include peroxides, such as diisopropyl peroxydicarbonate, benzoyl peroxide, t-butyl peroxyisobutyrate, t-hexyl peroxyisopropyl monocarbonate, t-hexylperoxy 2-ethylhexanoate, 1,1,3,3-tetramethylbutylperoxy 2-ethylhexanoate, t-butylperoxypivalate, t-hexyl peroxy pivalate, t-butyl peroxy neodecanoate, t-hexyl peroxy neodecanoate, 1,1,3,3-tetramethylbutylperoxyneodecanoate, 1,1-bis(t-hexylperoxy)cyclohexane, benzoyl peroxide, 3,5,5-trimethylhexanoyl peroxide, and lauroyl peroxide; and azo compounds, such as azobisisobutyronitrile, 2,2'-azobis(2-methylbutyronitrile), and dimethyl 2,2'-azobis(2-methylpropionate). These may be used in combination.

The thermal anionic polymerization initiator for use can be any commonly used thermal anionic polymerization initiator, without any particular limitation. Examples include amines and imidazoles. These may be used in combination.

Examples of amines include diethylenetriamine, triethylenetetramine, isophoronediamine, xylylenediamine, diaminodiphenylmethane, and 1,3,4,6-tetrakis(3-aminopropyl)glycoluril. Examples of imidazoles include 2-methylimidazole, 2-ethyl-4-methylimidazole, and 2-phenylimidazole.

The content of the thermal polymerization initiator in the second resin composition of the present invention is preferably 0.001 to 20% by weight, and more preferably 0.01 to 10% by weight, based on the entire second resin composition (total amount).

When the second resin composition of the present invention contains an epoxy resin (epoxy compound) as an additive (modifier), a photo-cationic polymerization initiator or a thermal cationic polymerization initiator may be used.

The photo-cationic polymerization initiator for use can be any commonly used photo-cationic polymerization initiator, without any particular limitation. Examples of photo-cationic polymerization initiators include onium salts and organic metal complexes.

Examples of onium salts include diazonium salts, sulfonium salts, and iodonium salts. Examples of organic metal complexes include iron-allene complexes, titanocene complexes, and aryl silanol-aluminum complexes.

Examples of commercially available photo-cationic photopolymerization initiators include Adeka Optomer SP-150 (trade name) and Adeka Optomer SP-170 (trade name) manufactured by Adeka Corporation, UVE-1014 (trade name) manufactured by General Electric Company, CD-1012 (trade name) manufactured by Sartomer, and CPI-100P (trade name) manufactured by San-Apro Ltd.

Examples of counter anions for photo-cationic polymerization initiators include SbF₆⁻, AsF₆⁻, B(C₆F₅)₄⁻, and PF₆⁻.

The thermal cationic polymerization initiator for use can be any commonly used thermal cationic polymerization initiator, without any particular limitation. Examples include various onium salts, such as quaternary ammonium salts, phosphonium salts, and sulfonium salts, and organic metal complexes. These may be used in combination.

Examples of commercially available onium salts include Adeka Opton CP-66 (trade name) and Adeka Opton CP-77 (trade name) produced by ADEKA Corporation, SunAid SI-60L (trade name), SunAid SI-80L (trade name), and SunAid SI-100L (trade name) produced by Sanshin Chemical Industry Co., Ltd., and CI series (trade name) produced by Nippon Soda Co., Ltd.

Examples of organic metal complexes include alkoxysilane-aluminum complexes.

The second resin composition of the present invention may further optionally contain the stabilizer, triphenylsilanol, talc, filler, calcium carbonate, additives (modifiers), and other components described in the First Resin Composition section above, as long as the effects of the present invention are not impaired. The additive (modifier) may be present in an amount of 0.01 to 50% by weight based on the entire second resin composition (total amount), as necessary.

The method for preparing (mixing) the second resin composition of the present invention is not particularly limited. For example, the second resin composition can be prepared by mixing the curing agent of the present invention, an ene compound, a photopolymerization initiator and/or a thermal polymerization initiator, optionally with an additional thiol compound and additives. The means for mixing components can be a known method (e.g., the methods described in the First Resin Composition section). The curing agent of the present invention (optionally with an additional thiol compound) may be dissolved or dispersed in a viscosity-adjusting diluent (organic solvent) beforehand.

### Use of Resin Composition

The first resin composition and the second resin composition of the present invention, each containing the curing agent of the present invention, (which may be collectively referred to as "the resin composition of the present invention") are expected to have low viscosity and give a cured product with excellent adhesive strength and mechanical strength.

Specifically, the resin composition of the present invention has a lower viscosity than known resin compositions containing thiol compounds, and gives a cured product with excellent adhesive strength and mechanical strength. Thus, the resin composition of the present invention can be suitably used as an adhesive, a sealant, an encapsulant, and a dam agent. That is, the adhesive and sealant of the present invention contain the above-described resin composition of the present invention as a component.

The adhesive and sealant of the present invention may contain additives. Examples of additives include fluid behavior modifiers, such as silicic acid, magnesium silicate, and barium sulfate; thermal-conductivity-imparting agents, such as alumina; electrical-conductivity-imparting agents, such as silver and carbon; and coloring agents, such as pigments and dyes. These additives can be added during preparation of the resin composition of the present invention. Alternatively, these additives may be mixed with the resin composition of the present invention prepared beforehand. The means for mixing additives can be a known method (e.g., the methods described in the First Resin Composition section).

The adhesive and sealant of the present invention are not particularly limited in terms of their applications and can be used in a wide range of areas. The adhesive can be used, for example, in adhesives for flexible printed-wiring boards; interlayer adhesives for multilayer boards such as build-up boards; adhesives for joining optical components; adhesives for bonding optical disks; adhesives for image sensors; adhesives used for mounting objects on printed-wiring boards; die bonding adhesives; adhesives for semiconductors such as underfills; mounting adhesives such as underfills for reinforcing BGAs, anisotropic conductive films (ACFs), and anisotropic conductive pastes (ACPs); adhesives for optical pickups; adhesives for optical path connection; adhesives used between exterior materials, base materials, or ceiling materials and interior materials; adhesives for bonding tiles and stone to exterior wall materials and base materials; adhesives for adhering wood flooring materials, polymeric floor sheets, or floor tiles to various floors; adhesives for structural materials, bodies, and parts of automobiles, aircraft, etc.; adhesives for automobile interiors; and adhesives for steel plate joints. The sealant can be used, for example, in sealants for joints in exterior materials such as various metal panels and siding boards; sealants used between exterior materials, base materials, or ceiling materials and interior materials; sealants for joints in various concrete products such as roads, bridges, tunnels, and breakwaters; sealants for structural materials, bodies, and parts of automobiles, aircraft, etc.; sealants for steel plate joints; and sealants for medical equipment.

With the recent miniaturization of electronic components and modules, adhesives and encapsulants for electronic components are sometimes injected into narrow areas using a jet dispenser. In such cases, the resin composition used in the adhesive or encapsulant is required to have a low viscosity. Because of its low viscosity, the resin composition of the present invention is particularly suitable for these applications.

The adhesive of the present invention also demonstrates excellent adhesion to engineering plastics, ceramics, and metals.

Engineering plastics refer to plastics that have a tensile strength of 49 MPa or more and a flexural modulus of elasticity of 2.5 GPa or more even under harsh conditions of 100°C and 100 hours.

Examples of engineering plastics include thermoplastic engineering plastics, such as polyacetal, polyamide, polycarbonate, modified polyphenylene ether, polybutylene terephthalate, glass fiber-reinforced polyethylene terephthalate, ultra-high-molecular-weight polyethylene, and syndiotactic polystyrene; thermosetting engineering plastics, such as epoxy, glass epoxy (FR-4), phenol, and silicone; and super engineering plastics, such as amorphous polyarylate, polysulfone, polyethersulfone, polyphenylene sulfide, polyether ether ketone, polyimide, polyetherimide, polyamide-imide, fluororesin, and liquid crystal polymers.

The super engineering plastics refer to engineering plastics that have a tensile strength of 49 MPa or more and a flexural modulus of elasticity of 2.5 GPa or more even under harsh conditions of 150°C and 100 hours.

These engineering plastics are suitable for use as a voice coil motor (VCM) in image sensor modules containing optical components.

Examples of ceramics include alumina, aluminum nitride, silicon carbide, silicon nitride, boron nitride, and glass. From the viewpoint of thermal conductivity, thermal expansion coefficient, and chemical durability, alumina and silicon nitride are preferred.

These ceramics are suitable for use as substrates for image sensor modules containing image pickup elements.

Examples of metals include stainless steel, nickel, nickel alloy, titanium, titanium alloy, copper, copper alloy, tin, tin alloy, aluminum, aluminum alloy, and solder. From the viewpoint of chemical stability such as oxidation resistance, stainless steel, nickel, and nickel alloy are preferred.

Table 1 shows an example of preferred formulations for the resin composition of the present invention used as an adhesive (in particular, a one-component adhesive).

**Table 1**

| | | Preferred Amount (Parts by Weight) |
|---|---|---|
| (A) Epoxy Compound(*) | | 100 |
| | Bisphenol A epoxy resin, Bisphenol F epoxy resin, 1,4-cyclohexanedimethanol diglycidyl ether, dicyclopentadiene dimethanol diglycidyl ether, or liquid epoxy resin with a naphthalene skeleton | |
| (B) Curing Agent(*) | | 10 to 200 |
| | 1,2,3-Tris(3-mercaptopropoxy)propane | |
| | Additional thiol compound (1,3,4,6-tetrakis(2-mercaptoethyl)glycoluril, or 1,3,4,6-tetrakis(3-mercaptopropyl)glycoluril) | 0 to 50 |
| (C) Curing Accelerator | | 1 to 80 |
| | Latent Curing Accelerator (Novacure HX-3088, Novacure HXA3922HP, Fujicure FXR-1030, or Fujicure FXR-1081) | |
| (D) Stabilizer | | 0 to 20 |
| | Triethyl borate,tri-n-propyl borate, triisopropyl borate, aluminum chelate, or barbituric acid | |
| (E) Filler | | 0 to 200 |
| | Silica Filler | |
| (F)Reactive Diluent | | 0 to 150 |
| | n-Butyl glycidyl ether, 2-ethylhexyl glycidyl ether, phenyl glycidyl ether, or allyl glycidyl ether | |
| (G) Additives | | 0 to 5 |
| | Glycidyl silane coupling agent or titanium coupling agent | |

| | | |
|---|---|---|
| (*) The components are added so that the ratio (equivalent ratio) of the number of thiol groups in component (B) to the number of epoxy groups in component (A) is 0.8 to 1.2. | | |

When used as a one-component adhesive, the resin composition of the present invention is applied to the area to be bonded and then thermally cured.

In regards to conditions for thermal curing, the heating temperature and heating time can be set appropriately. The heating temperature and heating time are preferably set to 80°C and 10 to 180 minutes, and more preferably 80°C and 30 to 60 minutes.

The resin composition of the present invention can be applied to products (parts and components) of various areas in which the materials can be resin, in addition to the adhesives and sealants described above. The resin composition can also be used as a raw material for materials in the areas of electricity or electronics, optics, construction, civil engineering, automobiles, aircraft, and medicine, as well as other daily necessities or miscellaneous goods.

Examples of parts, members, and materials in the electrical or electronic areas include resin-coated copper foil, prepreg, copper-clad laminates, printed-wiring boards, solder resist inks, conductive pastes, interlayer-insulating materials, encapsulants for LEDs, insulating materials, thermally conductive materials, hot melt materials, paints, and potting agents. More specific examples include encapsulating materials and layer-forming materials for printed-wiring boards and electronic components, such as interlayer-insulating films and wiring coating films; materials for forming display devices, such as color filters, films for flexible displays, resist materials, and alignment films; materials for forming semiconductor devices, such as resist materials and buffer coating films; and materials for forming optical components, such as holograms, optical waveguides, optical circuits, optical circuit components, and antireflection films.

Other examples include materials for forming rigid wiring boards and flexible printed-wiring boards for mounting semiconductors, materials for mounting semiconductors, encapsulants for semiconductors, encapsulants for solar cells, insulating films for semiconductors, coverlay films for protecting flexible printed-circuits, and coating agents for covering wiring.

Examples of materials in the optical area include core materials for optical fibers, cladding materials, lenses, and abrasion-resistant coating agents for lenses (e.g., hard-coat-forming solutions).

Examples of materials in the construction area include coating materials and primers for exterior materials, such as various metal panels and siding boards; injection materials used between exterior materials, base materials, or ceiling materials and interior materials, vibration-damping materials, soundproofing materials, conductive materials for electromagnetic wave shielding, and putty materials; adhesives for adhering wood flooring materials, polymeric floor sheets, or floor tiles to various floors; and injection materials for repairing cracks in various exterior and interior materials.

Examples of materials in the civil engineering area include coating materials, primers, paints, putty materials, injection materials, spraying materials, and casting materials for various concrete products, such as roads, bridges, tunnels, and breakwaters.

Examples of materials in the automotive or aircraft area include structural materials, coating materials for bodies and parts, cushioning materials, vibration-damping materials, soundproofing materials, and spraying materials; adhesives, coating materials, and foam materials for automotive interiors; and coating materials for steel plate joints.

Examples of materials in the medical area include artificial bones, dental impression materials, medical rubber materials, and medical adhesives.

### Examples

The present invention is described in more detail below with reference to Examples and Comparative Examples. However, the present invention is not limited to these Examples.

The main raw materials used are the following.

- 1,2,3-Triallyloxypropane (synthesized in accordance with the method described in JP2012-184198A. A compound represented by formula (II))
- Thioacetic acid (Tokyo Chemical Industry Co., Ltd.)
- Sulfuric acid (Wako Pure Chemical Industries, Ltd.)

### (A) Epoxy compound (epoxy resin)

- Bisphenol A epoxy resin (manufactured by Mitsubishi Chemical Corporation, trade name: jER828, epoxy equivalent: 187.0) (B) Curing agent
- Trimethylolpropane tris(3-mercaptopropionate) (manufactured by SC Organic Chemical Co., Ltd., trade name: TMMP, a compound represented by formula (VII), thiol equivalent: 133)
- Pentaerythritol tripropanethiol (manufactured by SC Organic Chemical Co., Ltd., trade name: PEPT, a compound represented by formula (VIII), thiol equivalent: 142)

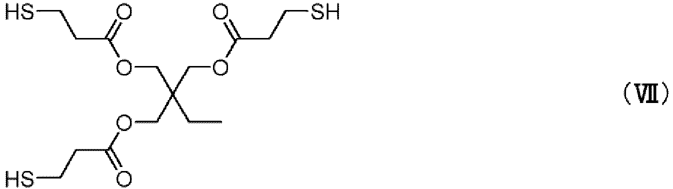

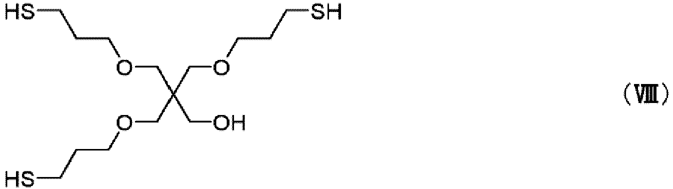

### (C) Curing Accelerator

- N-benzyldimethylamine (manufactured by Fujifilm Wako Pure Chemical Corporation)

The methods for measuring the viscosity, elastic modulus, and adhesive strength used in the evaluation tests in the Examples and Comparative Examples are as described below.

### Measurement of Viscosity

The viscosity of the compounds used as a curing agent and the epoxy resin composition was measured at 25°C with a viscometer (manufactured by UBM, Rheosol-G5000).

It is determined that the lower the viscosity (low viscosity), the higher the workability during the preparation of the epoxy resin composition and during the use of the resin composition as an adhesive.

### Measurement of Elastic Modulus

An epoxy resin composition was cured (80°C, 1 hour), and the obtained cured product (test specimen: length 25 mm × width 5 mm × thickness 1 mm) was measured for storage modulus of elasticity G' (MPa) or tensile modulus of elasticity E' (MPa) at 25°C with a dynamic viscoelastic meter (manufactured by UBM, Rheosol-G5000) (frequency: 1 Hz).

A smaller storage modulus of elasticity or a smaller tensile modulus of elasticity (low elasticity) is considered to indicate better impact resistance of the cured product.

### Measurement of Adhesive Strength (SUS)

An epoxy resin composition (adhesive) was applied to one side of each of two SUS plates (length 100 mm × width 25 mm × thickness 1.6 mm) in an area extending 12.5 mm from one end (longitudinal direction) (area: length 12.5 mm × width 25 mm).

Subsequently, the two SUS plates were laminated together so that the coated surfaces were in contact with each other and then heated (to cure the epoxy resin composition at 80°C for 1 hour), thereby preparing a test specimen. The tensile lap-shear strength (MPa) of the test specimen was measured in accordance with JIS K6850.

A higher shear strength is considered to indicate that the epoxy resin composition is more suitable as an adhesive.

### Measurement of Adhesive Strength (Al)

An epoxy resin composition (adhesive) was applied to one side of each of two blast-treated aluminum plates (length 100 mm × width 25 mm × thickness 1.6 mm) in an area extending 12.5 mm from one end (longitudinal direction) (area: length 12.5 mm × width 25 mm).

Subsequently, the two aluminum plates were laminated together so that the coated surfaces were in contact with each other and then heated (to cure the epoxy resin composition at 80°C for 1 hour), thereby preparing a test specimen.

The tensile lap-shear strength (MPa) of the test specimen was measured in accordance with JIS K6850 before and after high-temperature, high-pressure, steam treatment in an autoclave (PCT treatment, 121°C, 48 hours).

From the obtained measured values, the retention rate of tensile lap-shear strength after PCT treatment (which may be referred to as "strength retention rate" below) was calculated according to the following formula. Strength retention rate (%) = (tensile lap-shear strength after PCT treatment)/(tensile lap-shear strength before PCT treatment) × 100

A greater strength retention rate is considered to indicate better moisture resistance of the cured product, indicating that the epoxy resin composition is suitable for use as an adhesive.

### Synthesis of 1,2,3-(3-mercaptopropyloxy)propane Synthesis Example 1

A 500 mL three-necked recovery flask was charged with 50.95 g (240.0 mmol) of 1,2,3-triallyloxypropane and 152.85 g of butyl acetate and heated to 30°C. Thereafter, 60.28 g (792.0 mmol) of thioacetic acid was added dropwise, and the mixture was stirred at 30°C for 4 hours. Subsequently, the reaction solution was concentrated, and 1.17 g (12.0 mmol) of sulfuric acid and 307.58 g of methanol were added to the resulting reaction product, followed by stirring at 55°C for 30 hours. The resulting reaction solution was cooled, and butyl acetate and ion-exchanged water (butyl acetate/ion-exchanged water = 2/1 by weight) was added thereto at 20°C or lower, followed by washing with ion-exchanged water five times. The organic layer after washing with water was concentrated and purified by silica gel column chromatography (chloroform/toluene = 3/1 by volume), thereby obtaining 21.14 g of a colorless transparent liquid (column yield: 28.0%).

The ¹H-NMR spectrum data of the colorless transparent liquid were the following:
- ¹H-NMR (CDCl₃) δ: 3.69 (t, 2H), 3.56 (m, 5H), 3.50 (t, 4H), 2.64 (q, 6H), 1.87 (quin., 6H), 1.39 (t, 3H).

The IR spectrum data of the colorless transparent liquid was as shown in the chart of Fig. 1.

From these spectral data, the obtained colorless transparent liquid was identified as a thiol compound represented by formula (I) (1,2,3-(3-mercaptopropyloxy)propane).

### Evaluation of Epoxy Resin Composition

### Example 1

An epoxy resin composition was prepared by mixing 66.8 parts by weight of the thiol compound (thiol equivalent: 125.0) obtained in Synthesis Example 1 as a curing agent, 3.5 parts by weight of N-benzyldimethylamine as a curing accelerator, and 100.0 parts by weight of jER828 as an epoxy compound. The amount of the curing accelerator used was adjusted to 5% by weight relative to the curing agent.

The epoxy resin composition was subjected to evaluation tests (measurement of viscosity, measurement of storage modulus of elasticity of the cured product, and measurement of adhesive strength when used as an adhesive for SUS). The obtained test results were as shown in Table 2.

### Comparative Example 1

In the same manner as in Example 1, an epoxy resin composition of the formulation shown in Table 2 was prepared and subjected to evaluation tests. The obtained test results were as shown in Table 2.

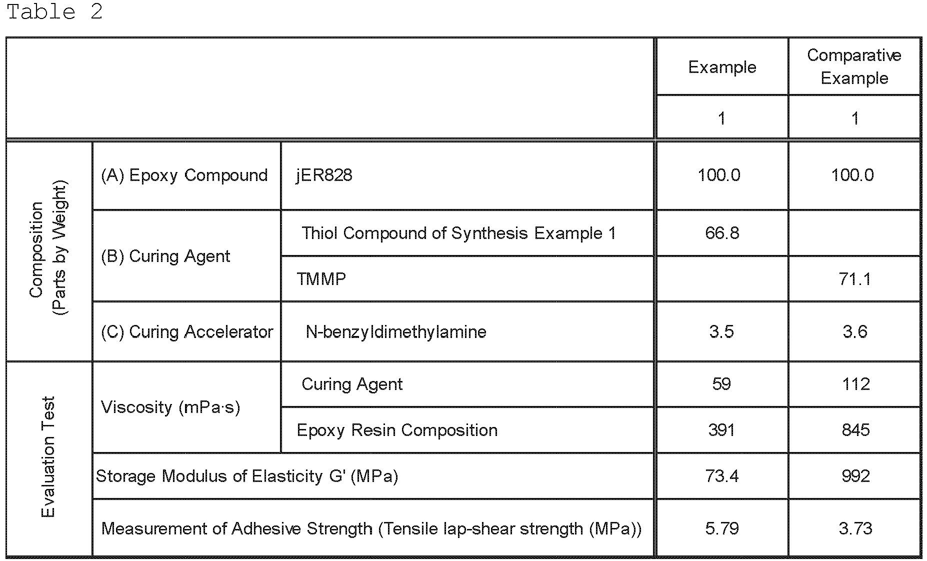

### Evaluation of Epoxy Resin Composition

### Example 2

An epoxy resin composition was prepared by mixing 66.8 parts by weight of the thiol compound obtained in Synthesis Example 1 as a curing agent, 1.3 parts by weight of N-benzyldimethylamine as a curing accelerator, and 100.0 parts by weight of jER828 as an epoxy compound. The amount of the curing accelerator used was adjusted to 2% by weight relative to the curing agent.

The epoxy resin composition was subjected to evaluation tests (measurement of the tensile modulus of elasticity of the cured product and measurement of the adhesive strength when used as an adhesive for Al). The obtained test results were as shown in Table 3. Additionally, the thiol compound obtained in Synthesis Example 1, used as a curing agent, was subjected to an evaluation test (measurement of viscosity), and the obtained test results were as shown in Table 3.

### Comparative Example 2

In the same manner as in Example 2, an epoxy resin composition of the formulation shown in Table 3 was prepared and subjected to evaluation tests. The obtained test results were as shown in Table 3. Additionally, PEPT, used as a curing agent, was subjected to an evaluation test (measurement of viscosity), and the obtained test results were as shown in Table 3.

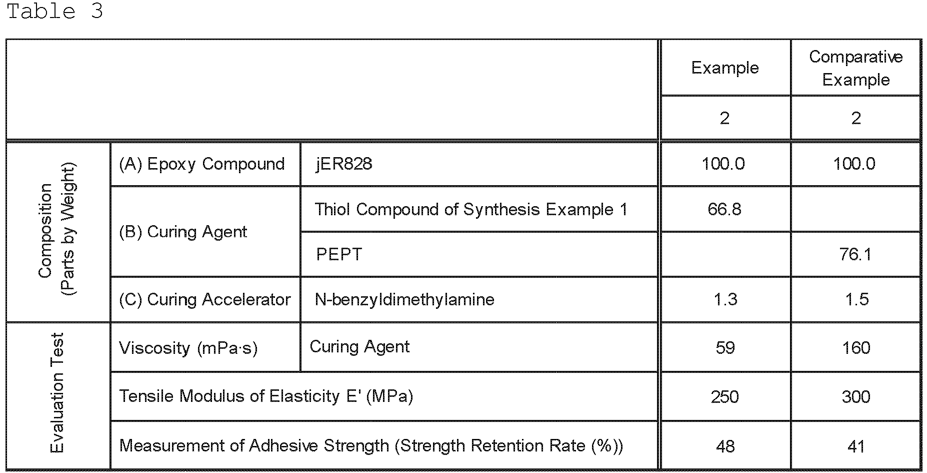

The results shown in Table 2 indicate that the use of the thiol compound of Synthesis Example 1 (1,2,3-tris(3-mercaptopropoxy)propane) as a curing agent (Example 1) resulted in excellence in handling due to a lower viscosity of the curing agent and the epoxy resin composition, excellence in impact resistance due to a lower storage modulus of elasticity of the cured product, and excellence as an adhesive due to a higher shear strength, as compared with the use of TMMP (Comparative Example 1).

The results shown in Table 3 indicate that the use of the thiol compound of Synthesis Example 1 (1,2,3-tris(3-mercaptopropoxy)propane) as a curing agent (Example 2) resulted in excellence in handling due to a lower viscosity of the curing agent, excellence in impact resistance due to a lower tensile modulus of elasticity of the cured product, and excellence as an adhesive due to a higher adhesive strength retention rate after high-temperature, high-pressure steam treatment, as compared with the use of PEPT (Comparative Example 2).

### Industrial Applicability

The curing agent containing the thiol compound represented by formula (I) of the present invention is useful as a curing agent for resins such as epoxy resins. Additionally, the resin composition containing the curing agent, an epoxy compound, and optional other components is suitable for various applications such as in adhesion, sealing, encapsulation, casting, molding, painting, and coating.

## Claims

1. A curing agent comprising a thiol compound represented by formula (I): 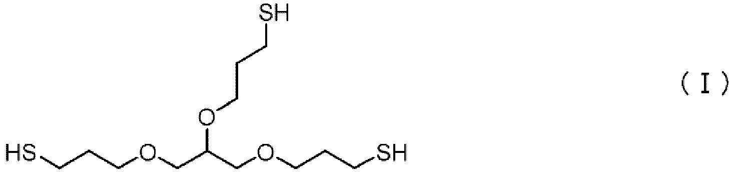 .

2. A resin composition comprising the curing agent of claim 1 and an epoxy compound.

3. The resin composition according to claim 2, further comprising an amine as a curing accelerator.

4. A resin composition comprising the curing agent of claim 1 and an ene compound having a carbon-carbon double bond in a molecule.

5. An adhesive comprising the resin composition of any one of claims 2 to 4.

6. A sealant comprising the resin composition of any one of claims 2 to 4.

7. A cured product of the resin composition of any one of claims 2 to 4.
